# EUROPEAN PATENT APPLICATION

(11) **EP 4 487 766 A1**
(43) Date of publication of application: **08.01.2025**
(21) Application number: 22958491.7
(22) Date of filing: 16.09.2022
(51) Int. Cl.: A61B 5/053

(54) **SIGNAL MEASUREMENT CIRCUIT AND METHOD**

(71) Applicant: Shenzhen Shokz Co., Ltd., Shenzhen, Guangdong 518108 (CN)
(72) Inventor: ZHOU, Xin, Shenzhen, Guangdong 518108 (CN); ZHANG, Yuxiang, Shenzhen, Guangdong 518108 (CN); SU, Lei, Shenzhen, Guangdong 518108 (CN); LI, Meiqi, Shenzhen, Guangdong 518108 (CN); LIU, Jia, Shenzhen, Guangdong 518108 (CN); LIAO, Fengyun, Shenzhen, Guangdong 518108 (CN); QI, Xin, Shenzhen, Guangdong 518108 (CN)
(74) Representative: Wang, Bo
(86) International application number: PCT/CN2022/119380
(87) International publication number: WO 2024/055301

(57) **Abstract**

The embodiments of the present disclosure disclose a signal measurement circuit and method. The signal measurement circuit include: a plurality of electrodes configured to be attached to a human body and collect physiological signals of the human body; a contact impedance detection circuit electrically connected with the plurality of electrodes, the contact impedance detection circuit being configured to measure a contact impedance between each of the plurality of electrodes and the human body; and a switching circuit configured to control a conduction state between the plurality of electrodes and the contact impedance detection circuit, such that only a portion of the plurality of electrodes are in the conduction state with the contact impedance detection circuit simultaneously. By measuring the contact impedances between the plurality of electrodes and the human body and determining an attachment state between the plurality of electrodes and the human body based on the contact impedances, the quality of physiological signals collected by the plurality of electrodes can be ensured. By selecting different electrodes to be in the conduction state with the contact impedance detection circuit at different times to collect the contact impedances corresponding to different portions of electrodes in a time-sharing manner, the quality of the collected physiological signals can be ensured.

## Description

### TECHNICAL FIELD

The present disclosure relates to the field of circuit design, and in particular, to a signal measurement circuit and a signal measurement method.

### BACKGROUND

As people pay more and more attention to the fields of physiological monitoring, disease diagnosis, experimental research, etc., the research on the circuit structure of signal measurement is also becoming more and more important. A signal measurement circuit can often obtain data related to the user's physical condition by collecting physiological signals. For example, the signal measurement circuit can monitor and use the information of electromyographic signals to make clinical diagnoses of various diseases such as muscle strength, muscle fatigue, and even myasthenia gravis, myotonia, and muscular atrophy.

However, when measuring physiological signals using a signal acquisition circuit, poor adhesion between the electrodes and the human body, as well as the skin being nearly insulating in dry environments, often result in low-quality physiological signals, making accurate monitoring difficult.

Therefore, it is desirable to provide a device capable of collecting physiological signals with improved quality.

### SUMMARY

One of the embodiments of the present disclosure provides a signal measurement circuit. The signal measurement circuit may include: a plurality of electrodes configured to be attached to a human body and collect physiological signals of the human body; a contact impedance detection circuit electrically connected with the plurality of electrodes, the contact impedance detection circuit being configured to measure a contact impedance between each of the plurality of electrodes and the human body; and a switching circuit configured to control a conduction state between the plurality of electrodes and the contact impedance detection circuit, such that only a portion of the plurality of electrodes is in the conduction state with the contact impedance detection circuit simultaneously.

In some embodiments, the plurality of electrodes may include two electrodes attached to the same muscle. The two electrodes attached to the same muscle may be synchronously in the conduction state with the contact impedance detection circuit.

In some embodiments, the plurality of electrodes may include two electrodes attached to the same muscle. The two electrodes attached to the same muscle may be in the conduction state with the contact impedance detection circuit at different times.

In some embodiments, the contact impedance detection circuit may include: an excitation source configured to provide an excitation signal to the two electrodes attached to the same muscle to generate detection signals each of which corresponds to one of the two electrodes attached to the same muscle, each detection signal reflecting the contact impedance between the corresponding electrode and the human body; two voltage-dividing impedances, each of the two voltage-dividing impedances being coupled between the excitation source and one of the two electrodes attached to the same muscle to form the corresponding detection signal by performing a voltage division on the excitation source; and two analog-to-digital converters configured to perform an analog-to-digital conversion on the detection signals, respectively.

In some embodiments, the contact impedance detection circuit may include: an excitation source configured to provide an excitation signal to each of the plurality of electrodes to generate a detection signal reflecting the contact impedance between each of the plurality of electrodes and the human body; a voltage-dividing impedance coupled between the excitation source and each of the plurality of electrodes to form the detection signal by performing a voltage division on the excitation source; and an analog-to-digital converter configured to perform an analog-to-digital conversion on the detection signal.

In some embodiments, the excitation source may be a DC excitation source or an AC excitation source with a frequency not less than 0.1 Hz.

In some embodiments, the voltage dividing impedance may include a resistor or a capacitor.

In some embodiments, a follower may be coupled between the analog-to-digital converter and the voltage dividing impedance.

In some embodiments, the signal measurement circuit may further include a gain circuit configured for processing the physiological signals. The gain circuit and the contact impedance detection circuit may be in the conduction state with the plurality of electrodes at different times, respectively.

In some embodiments, the signal measurement circuit may further include a processing circuit configured to determine parameter information reflecting an attachment state of each of the plurality of electrodes to the human body according to the contact impedance of each of the plurality of electrodes.

One of the embodiments of the present disclosure provides a method for signal measurement. The method for signal measurement may include: controlling a conduction state between a plurality of electrodes and a contact impedance detection circuit through a switching circuit, such that only a portion of the plurality of electrodes are in the conduction state with the contact impedance detection circuit simultaneously, wherein the plurality of electrodes may be configured to be attached to a human body and collect physiological signals of the human body, and the contact impedance detection circuit may be configured to measure a contact impedance between each of the plurality of electrodes and the human body; and determining parameter information reflecting an attachment state of each of the plurality of electrodes to the human body according to the contact impedance of each of the plurality of electrodes.

In some embodiments, the method for signal measurement may further include: determining a warm-up state of a user based on a difference or a ratio of the contact impedances of two of the plurality of electrodes attached to the same muscle.

In the embodiments of the present disclosure, the contact impedances between the plurality of electrodes and the human body are measured, and the attachment state between the plurality of electrodes and the human body is determined based on the contact impedances, thereby ensuring the quality of the physiological signals collected by the plurality of electrodes.

Furthermore, the embodiments of the present disclosure can also select different portions of electrodes to be in the conduction state with the contact impedance detection circuit at different times to collect the contact impedances corresponding to different electrodes in a time-sharing manner, thereby ensuring the quality of the physiological signals collected by different electrodes of the signal measurement circuit.

### BRIEF DESCRIPTION OF THE DRAWINGS

The present disclosure will be further illustrated by way of exemplary embodiments, which will be described in detail by means of the accompanying drawings. These embodiments are not limiting, and in these embodiments, the same numbering indicates the same structure, wherein:
FIG. 1 is a structural block diagram illustrating an exemplary signal measurement circuit according to some embodiments of the present disclosure;
FIG. 2 is a structural block diagram illustrating an exemplary contact impedance detection circuit according to some embodiments of the present disclosure;
FIG. 3 is a schematic diagram illustrating an exemplary signal measurement circuit according to some embodiments of the present disclosure;
FIG. 4 is a structural block diagram illustrating an exemplary signal measurement circuit according to other embodiments of the present disclosure;
FIG. 5 is a schematic structural diagram illustrating an exemplary signal measurement circuit according to some embodiments of the present disclosure;
FIG. 6 is a schematic diagram illustrating exemplary curves of contact impedances corresponding to a plurality of electrodes during exercise of a user according to some embodiments of the present disclosure; and
FIG. 7 is a flowchart illustrating an exemplary process for signal measurement according to some embodiments of the present disclosure.

### DETAILED DESCRIPTION

In order to more clearly illustrate the technical solutions of the embodiments of the present disclosure, the accompanying drawings required to be used in the description of the embodiments are briefly described below. Obviously, the accompanying drawings in the following description are only some examples or embodiments of the present disclosure, and it is possible for a person of ordinary skill in the art to apply the present disclosure to other similar scenarios in accordance with these drawings without creative labor. Unless obviously obtained from the context or the context illustrates otherwise, the same numeral in the drawings refers to the same structure or operation.

It should be understood that the terms "system", "device", "unit" and/or "module" used herein are a way to distinguish between different components, elements, parts, sections, or assemblies at different levels. However, the terms may be replaced by other expressions if other words accomplish the same purpose.

As shown in the present disclosure and in the claims, unless the context clearly suggests an exception, the words "one", "a", "an", "one kind", and/or "the" do not refer specifically to the singular, but may also include the plural. Generally, the terms "including" and "comprising" suggest only the inclusion of clearly identified steps and elements, however, the steps and elements that do not constitute an exclusive list, and the method or apparatus may also include other steps or elements.

Flowcharts are used in the present disclosure to illustrate the operations performed by a system according to embodiments of the present disclosure, and the related descriptions are provided to aid in a better understanding of the magnetic resonance imaging method and/or system. It should be appreciated that the preceding or following operations are not necessarily performed in an exact sequence. Instead, steps can be processed in reverse order or simultaneously. Also, it is possible to add other operations to these processes or to remove a step or steps from these processes.

The signal measurement circuit and method provided by one or more embodiments of the present disclosure may be applied to a device for collecting one or more physiological signals, such as a smart wearable device, a medical detection device, or a signal analysis device. In some embodiments, the smart wearable device may be set at various parts (e.g., lower legs, upper legs, the waist, the back, the chest, shoulders, the neck, etc.) of a human body to collect the physiological signals from various parts of the user's body in different states. The collected physiological signals may be further processed. In some embodiments, the smart wearable device may include a smart bracelet, smart shoes and socks, smart glasses, a smart helmet, a smart watch, smart clothing, a smart backpack, a smart accessory, or the like, or any combination thereof.

In some embodiments, a physiological signal refers to a detectable signal that reflects the state of the body. For example, the physiological signal may include a respiratory signal, an electrocardiogram (ECG), an electromyographic signal, a blood pressure signal, a temperature signal, etc. In some embodiments, a frequency of the physiological signal may be within a range of 0.05 Hz-2 kHz. A frequency of the ECG may be within a range of 0.05 Hz-100 Hz. A frequency of the electromyographic signal may be within a range of 10 Hz-850 Hz.

In some embodiments, the intensity of the physiological signal collected by the signal measurement circuit may be affected by various factors, such as contact impedances between the human body and the electrodes, an activation degree of a human muscle, etc. The contact impedance refers to an impedance generated by the contact between the human skin and an electrode of the signal measurement circuit, and the impedance value is affected by the dryness of the skin and the attachment state between the human body and the electrodes, thereby affecting the quality of the physiological signals. For example, the drier the skin, the greater the impedance value of the contact impedance, the worse the quality of the collected physiological signal; conversely, the wetter the skin, the smaller the impedance value of the contact impedance, and the better the quality of the collected physiological signal. As another example, poor contact between the human body and the electrodes causes the impedance value of the contact impedance to increase, making the quality of the physiological signals worse.

In some embodiments, the activation degree of the human muscle refers to an active state of the muscle at work, which can affect the intensity of the electromyographic signal released by the muscle. Users have various daily exercise monitoring needs (such as walking and standing), which, compared to fitness monitoring needs (like squats and running), involve lower muscle activation. This results in weaker physiological signal recognition (such as electromyography signals), affecting subsequent identification and processing. In this case, if the user does not wear the electrodes correctly or the user's skin is too dry during the collection process of the physiological signals, the contact impedances between the human body and the electrodes may be relatively large, resulting in poor quality of the collected physiological signals, and making it difficult to realize accurate physiological monitoring for the user.

Therefore, according to some embodiments of the present disclosure, the contact impedances between the electrodes and the human body may be measured by a contact impedance detection circuit, and the attachment state between the electrodes and the human body may be determined based on the contact impedances, thereby ensuring the quality of the physiological signals collected by the electrodes. Furthermore, when it is necessary to monitor the attachment state between the plurality of electrodes and different parts of the human body, different portions of electrodes may be selected to be in the conduction state with the contact impedance detection circuit at different times, so as to collect the contact impedances corresponding to different portions of electrodes in a time-sharing manner, thereby preventing an interference between different electrodes and saving resources of the contact impedance detection circuit.

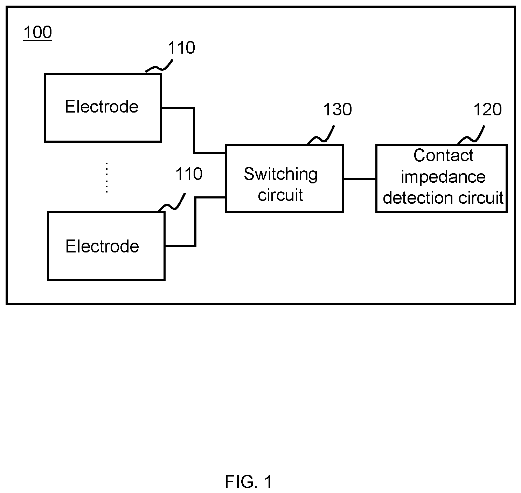

FIG. 1 is a structural block diagram illustrating an exemplary signal measurement circuit according to some embodiments of the present disclosure. As shown in FIG. 1, a signal measurement circuit 100 may include a plurality of electrodes 110, a contact impedance detection circuit 120, and a switching circuit 130. The plurality of electrodes 110 may be electrically connected with the switching circuit 130. The contact impedance detection circuit 120 may be electrically connected with the switching circuit 130. In some embodiments, the plurality of electrodes 110 may be configured to be attached to a human body and collect physiological signals of the human body. The contact impedance detection circuit 120 may be configured to measure a contact impedance between each of the plurality of electrodes 110 and the human body. The switching circuit 130 may be configured to control a conduction state between the plurality of electrodes 110 and the contact impedance detection circuit 120. In some embodiments, only a portion of the plurality of electrodes 110 is in the conduction state with the contact impedance detection circuit 120 simultaneously.

The plurality of electrodes 110 may be circuit elements that input or output current through contact. In some embodiments, the plurality of electrodes 110 may be attached to the human body to collect physiological signals (e.g., an electromyographic signals, an ECG signal, a blood pressure signal, etc.) of the human body. In some embodiments, the plurality of electrodes 110 may collect physiological signals of the human body within a target frequency range. The target frequency range may include frequencies of most physiological signals generated by the human body, and may also include frequencies of physiological signals that are expected to be obtained.

In some embodiments, the plurality of electrodes 110 may be attached to different parts of the human body to collect physiological signals from the different parts. For example, the plurality of electrodes 110 may be attached to the user's chest, wrist, back, leg, arm, head, etc. In some embodiments, one of the plurality of electrodes may be configured to collect physiological signals from one part, or two of the plurality of electrodes may be used as an electrode group to collect physiological signals from the same part.

In some embodiments, the plurality of electrodes 110 may include two electrodes 110 attached to the same muscle. In some embodiments, the two electrodes 110 attached to the same muscle may be used as a group of electrodes to collect two physiological signals of the same muscle at the same time. In some embodiments, the two physiological signals at the same time may be subjected to differential, summation, and other calculation processes to form parameters reflecting a movement state of the same muscle. In this way, inaccurate measurement of physiological signals due to damage to a certain electrode or poor attachment to the human body may be prevented. In some embodiments, the two electrodes 110 attached to the same muscle may also work in a time-sharing manner to collect the two physiological signals from the part at different times. The two physiological signals at different times may reflect changes in the physiological signals to monitor a physiological state of the user. More descriptions regarding working in a time-sharing manner may be elsewhere in the present disclosure (e.g., the switching circuit and related descriptions thereof).

In some embodiments, the contact impedance between each of the plurality of electrodes and the human body may generate a detection signal under an external excitation (e.g., an excitation signal), such that the contact impedance corresponding to each of the plurality of electrodes may be determined according to the detection signal. In some embodiments, the detection signal refers to a signal reflecting a magnitude of the contact impedance. Accordingly, the detection signal may reflect the quality of the physiological signal. By analyzing the detection signal corresponding to a single electrode 110 or a difference between the detection signals corresponding to the plurality of electrodes, the quality of the physiological signal(s) collected by the single electrode or the plurality of electrodes may be evaluated. For example, if the detection signal corresponding to a certain electrode indicates that the contact impedance between the electrode and the human body is greater than a certain threshold, it means that the electrode is not worn correctly or the user's skin is too dry. As another example, if the difference between the detection signals corresponding to two electrodes is large, it means that the difference in the contact impedances between the two electrodes and the human body is large, indicating that at least one of the two electrodes is not worn correctly or the user's skin is too dry, and the quality of the collected physiological signals is poor. Conversely, if the difference between the detection signals corresponding to the two electrodes is small, it means that the difference in the contact impedances between the two electrodes and the human body is small, indicating that the quality of the collected physiological signals is good. In some embodiments, the detection signal may also reflect other information such as a user's wearing state of electrodes, body composition information, and a skin condition.

In some embodiments, the contact impedances corresponding to the two electrodes 110 attached to the same muscle may generate two detection signals at the same time, and attachment states of the two electrodes 110 to the human body at the same time may be recognized according to the two detection signals, thereby reflecting the quality of the physiological signals collected at that time. In some embodiments, external excitations corresponding to the two electrodes 110 may provide excitation signals of different frequencies. After performing signal processing on the detection signals of different frequencies, the impedance information of the human body at different frequencies may be obtained, thereby reflecting the body composition information of the human body. In some embodiments, the two electrodes 110 attached to the same muscle may generate two detection signals at different times. In some embodiments, an interval between acquisition times of the two detection signals may be very short, and during this period, it can still be considered that the attachment states of the two electrodes 110 to the human body do not change. The two detection signals may be processed, for example, through differential or summation operations, which may still reflect the attachment states of the two electrodes 110 to the human body during this period, thereby reflecting the quality of the physiological signals during the same period.

In some embodiments, the plurality of electrodes 110 may be in contact with the human body in the form of flexible patches. The flexible patches may have a regular shape such as a circle, an ellipse, a rectangle, a diamond, or other irregular shapes. In some embodiments, the plurality of electrodes may be made of a metal conductive material (e.g., copper, aluminum, nickel, chromium, an alloy, etc.), a non-metal conductive material (e.g., conductive plastic, conductive rubber, conductive silicone, a conductive fabric), or other conductive materials.

In some embodiments, the signal measurement circuit 100 may include one or more reference ground electrodes (not shown in FIG. 1). The one or more reference ground electrodes may be used as reference grounds in the signal measurement circuit 100. In some embodiments, the one or more reference ground electrodes may be configured to be attached to the human body such that the excitation signals may flow to the one or more reference ground electrodes through the human body to form a closed loop. In some embodiments, the one or more reference ground electrodes may be set in a flat and relatively stable (less moving) part, such as the back of the human body, the head of the human body, etc., so as to effectively reduce noise.

The contact impedance detection circuit 120 refers to a structural module having a certain signal processing function (e.g., amplification, rectification, A/D conversion, filtering, logic processing, etc.). In some embodiments, the contact impedance detection circuit 120 may determine the contact impedance between each of the plurality of electrodes 110 and the human body based on the detection signal corresponding to each of the plurality of electrodes.

Merely by way of example, in some embodiments, the contact impedance detection circuit 120 may process the detection signal corresponding to a certain electrode 110 separately, and evaluate the physiological signal collected by the electrode based on the detection signal. For example, the contact impedance detection circuit 120 may perform an analog-to-digital (A/D) conversion on the detection signal corresponding to the electrode 110, and a converted digital signal may reflect the contact impedance between the electrode 110 and the human body separately after further processing, thereby reflecting the attachment state of the electrode 110 to the human body, and determining the quality of the physiological signal collected by the corresponding electrode 110.

In some embodiments, the contact impedance detection circuit 120 may comprehensively evaluate the physiological signals collected by the plurality of electrodes 110 based on the detection signals corresponding to the plurality of electrodes 110. For example, the contact impedance detection circuit 120 may perform an operation (e.g., differential, and summation) on the detection signals corresponding to the plurality of electrodes 110 (e.g., the two electrodes 110 attached to the same muscle), and then perform the analog-to-digital (A/D) conversion on the result of the operation. The detection signals after the operation may comprehensively reflect the difference in the contact impedances between the two electrodes 110 and the human body, thereby reflecting the attachment states of the two electrodes 110 to the human body, and determining the quality of the physiological signals.

In some embodiments, if the contact impedance detection circuit 120 is in the conduction state with the plurality of electrodes 110, data transmission with the plurality of electrodes 110 may be performed. The contact impedance detection circuit 120 may simultaneously obtain the detection signals corresponding to the plurality of electrodes 110 and the physiological signals collected by the plurality of electrodes 110. In some embodiments, the contact impedance detection circuit 120 may also filter the detection signals corresponding to the plurality of electrodes 110 to distinguish the detection signals corresponding to the plurality of electrodes 110 from the physiological signals collected by the plurality of electrodes 110. If the frequencies of the excitation signals are different from the frequencies of the physiological signals, the frequencies of the detection signals generated by the excitation signals may also be different from the frequencies of the physiological signals, such that the detection signals and the physiological signals may be extracted by a filter, respectively, to evaluate the physiological signals and the detection signals subsequently. Since the intensity of the detection signals corresponding to the plurality of electrodes 110 is greater than the intensity of the physiological signals collected by the plurality of electrodes 110, in some embodiments, the contact impedance detection circuit 120 may distinguish the detection signals corresponding to the plurality of electrodes 110 from the physiological signals collected by the plurality of electrodes 110 based on the signal intensity through a follower and an analog-to-digital converter.

In some embodiments, one contact impedance detection circuit 120 may be provided. The contact impedance detection circuit 120 may be coupled with the switching circuit 130. In this case, the switching circuit 130 may select one or more of the plurality of electrodes 110 to be conducted with the contact impedance detection circuit 120 at different times.

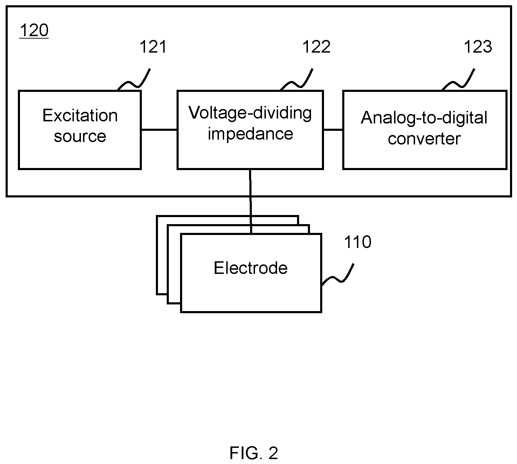

In some embodiments, a plurality of contact impedance detection circuits 120 may be provided. In this case, the switching circuit 130 may select a portion of the plurality of contact impedance detection circuits 120 to be in the conduction state with a portion of the plurality of electrodes 110. That is, the switching circuit 130 may simultaneously select the electrodes 110 that need to be conducted and the contact impedance detection circuits 120 that need to be conducted. In some embodiments, in response to determining that the plurality of contact impedance detection circuits 120 are provided, a plurality of switching circuits 130 may be provided. Each of the plurality of contact impedance detection circuits 120 may be coupled with one or more electrodes 120 through one of the plurality of switching circuits 130. The plurality of switching circuits 130 may control the plurality of contact impedance detection circuits 120 to be in the conduction state with the plurality of electrodes at the same time. That is, the plurality of switching circuits 130 may control the plurality of electrodes 110 to be conducted with the plurality of contact impedance detection circuits 120 corresponding to the plurality of electrodes 110. More descriptions regarding the contact impedance detection circuit 120 may be found elsewhere in the present disclosure (e.g., FIG. 2 and related descriptions thereof).

The switching circuit 130 refers to a control structure for switching between a conduction state and an off state of a plurality of devices. In some embodiments, the conduction state may reflect that data can be transmitted between the plurality of devices. The off state may reflect that data is difficult to be transmitted between the plurality of devices. In some embodiments, an input end of the switching circuit 130 may be connected with the plurality of electrodes 110, and an output end of the switching circuit 130 may be connected with the contact impedance detection circuit 120. The switching circuit 130 may control the switching between the conduction state and the off state of the input end and the output end. The switching circuit 130 may select the input end corresponding to one or more electrodes 110 of the plurality of electrodes 110 to be in the conduction state with the output end of the switching circuit 130, so that the one or more electrodes 110 of the plurality of electrodes 110 may be in the conduction state with the contact impedance detection circuit 120.

In some embodiments, the switching circuit 130 may control a portion of the plurality of electrodes 110 to be in the conduction state at the same time, so as to synchronously measure contact impedances corresponding to the portion of the plurality of electrodes 110 in the conduction state. In this case, the contact impedance detection circuit 120 may measure the contact impedances corresponding to the portion of the electrodes 110 and the contact impedances corresponding to other electrodes 110 in a time-sharing manner. In some embodiments, the switching circuit 130 may control each of the plurality of electrodes 110 to be in the conduction state in a time-sharing manner, so as to measure the contact impedance corresponding to each of the plurality of electrodes 110. In some embodiments, the switching circuit 130 may control the plurality of electrodes 110 to collect the physiological signals and measure the contact impedances corresponding to the plurality of electrodes 110 in a time-sharing manner. The above three time-sharing measurements are described below.

In some embodiments, the switching circuit 130 may control different portions of the electrodes 110 to be in the conduction state with the contact impedance detection circuit 120 at different times, so as to measure the contact impedances between different portions of electrodes and the human body in a time-sharing manner. For example, the plurality of electrodes 110 may include two electrodes 110 attached to the same muscle, and the switching circuit 130 may control the two electrodes 110 attached to the same muscle to be synchronously in the conduction state with the contact impedance detection circuit 120. In this case, the contact impedance detection circuit 120 may synchronously detect the contact impedance between each of the two electrodes 110 and the human body, so as to determine the quality of the physiological signals (e.g., the electromyographic signals) collected by the two electrodes 110. That is, two of the plurality of electrodes 110 may be selected to be measured at the same time, and other electrodes 110 may be measured at one or more other times. The contact impedance detection circuit 120 may simultaneously obtain the detection signals corresponding to the two electrodes 110 attached to the same muscle, such that the contact impedance between each of the two electrodes 110 and the human body may be synchronously detected to evaluate the quality of the physiological signals corresponding to the muscle. In some embodiments, the contact impedance detection circuit 120 may also determine the contact impedance corresponding to each of the two electrodes 110 according to the two detection signals, so as to evaluate the quality of the physiological signals collected by the two electrodes 110, respectively. In some embodiments, if the quality of the two electromyographic signals collected by the two electrodes 100 attached to the same muscle is good, operational amplification (e.g., differential amplification) may be further performed on the two electromyographic signals by other processing circuits to obtain parameter values reflecting the state of the muscle. In some embodiments, the other processing circuits may calibrate or modify the two electromyographic signals according to the contact impedances between the two electrodes 110 and the human body, thereby improving the accuracy of the electromyographic signals collected by the electrodes.

In this way, the switching circuit 130 may capture the contact impedance information of the two electrodes attached to the same muscle at the same time by controlling the two electrodes 110 attached to the same muscle to be synchronously in the conduction state with the contact impedance detection circuit 120, thereby avoiding the fluctuation in the contact impedance over time and the interference from other signals (e.g., the detection signals corresponding to other electrodes), and more accurately evaluating the quality of the physiological signals.

In some embodiments, the two electrodes 110 attached to the same muscle may be used as an electrode group. The plurality of electrodes 110 may be divided into a plurality of electrode groups. The switching circuit 130 may control one or more electrode groups to be in the conduction state with the contact impedance detection circuit 120 to simultaneously measure the contact impedance. Merely by way of example, assuming that four electrode groups are provided, the switching circuit 130 may control the two electrodes 110 in one of the four electrode groups to be simultaneously in the conduction state with the contact impedance detection circuit 120, while controlling other three electrode groups of the four electrode groups to be in the off state and not to perform data transmission with the contact impedance detection circuit 120. As another example, the switching circuit 130 may control the electrodes 110 in two of the four electrode groups to be simultaneously in the conduction state with the contact impedance detection circuit 120, while controlling other two electrode groups of the four electrode groups to be in the off state and not to perform data transmission with the contact impedance detection circuit 120, thereby obtaining the contact impedance information of the two electrode groups at the same time.

In some embodiments, the switching circuit 130 may control each of the plurality of electrodes 110 to be in the conduction state with the contact impedance detection circuit 120 at different times, so as to measure the contact impedance between each of the plurality of electrodes 110 and the human body in a time-sharing manner. For example, the switching circuit 130 may control two electrodes 110 attached to the same muscle to be in the conduction state with the contact impedance detection circuit 120 at different times. In this case, the contact impedance detection circuit 120 may measure the contact impedances between the two electrodes 110 and the human body in a time-sharing manner, so as to determine the quality of the physiological signals (e.g., the electromyographic signals) collected by the two electrodes 110. In other words, the contact impedance detection circuit 120 may asynchronously obtain the detection signals corresponding to the two electrodes 110 to evaluate the quality of the physiological signals corresponding to the muscle. In some embodiments, the contact impedance detection circuit 120 may determine the contact impedance corresponding to each of the two electrodes 110 based on the two detection signals, so as to evaluate the quality of the physiological signals collected by the two electrodes 110, respectively. Since the interval between the acquisition times of the two detection signals is very short, in some embodiments, the contact impedance detection circuit 120 may also determine contact impedances difference between the two electrodes 110 based on a difference between the two detection signals to evaluate the quality of the physiological signals collected by the two electrodes 110.

In this way, the contact impedance detection circuit 120 may asynchronously detect the contact impedances between the two electrodes 110 and the human body, reducing the burden of subsequent circuits in processing the detection signals at the same time (e.g., reducing the requirements of a sampling frequency in a subsequent process of analog-to-digital conversion). In addition, asynchronous detection may save a subsequent circuit cost and prevent a mutual interference between the two electrodes 110, thereby further improving the quality of the physiological signals.

Merely by way of example, the contact impedance detection circuit 120 may obtain a detection signal corresponding to a first electrode at a first time, then wait until a second time to obtain a detection signal corresponding to a second electrode, and evaluate the quality of the physiological signals collected by the two electrodes 110 according to the detection signal corresponding to the first electrode and the detection signal corresponding to the second electrode. It should be noted that in order to ensure the signal stability in a switching channel and prevent the signal error caused by asynchronous detection, an interval between the first time and the second time may be set very short. If the excitation source is an AC excitation source, since the contact impedance detection circuit 120 needs to wait for a complete cycle to read a plurality of detection signals, in some embodiments, the interval between the first time and the second time may also be set according to a frequency of the AC excitation source. For example, the interval between the first time and the second time may be 100 µs, 1 ms, 10 ms, 20 ms, etc.

In some embodiments, if a plurality of contact impedance detection circuits 120 are provided, the switching circuit 130 may control a portion of the plurality of contact impedance detection circuits 120 to be in the conduction state, and another portion of the contact impedance detection circuits 120 to be in the off state, such that the portion of the contact impedance detection circuits 120 that need to be in the conduction state may perform data transmission with the portion of the electrodes 110 that need to be in the conduction state. In some embodiments, the plurality of contact impedance detection circuits 120 may correspond to the plurality of electrodes 110, and the switching circuit 130 may select the contact impedance detection circuits 120 corresponding to the portion of the electrodes 110, such that the portion of the electrodes 110 may be in the conduction state with the contact impedance detection circuits 120 corresponding to portion of the electrodes 110.

Merely by way of example, each of the plurality of electrodes 110 may correspond to one contact impedance detection circuit 120, and the switching circuit 130 may control each of the plurality of electrodes 110 to be in the conduction state with the contact impedance detection circuit 120 corresponding to the electrode 110 in a time-sharing manner. As another example, each electrode group may correspond to one contact impedance detection circuit 120, and the switching circuit 130 may control each of different electrode groups to be in the conduction state with the contact impedance detection circuit 120 corresponding to the electrode group in a time-sharing manner.

In some embodiments, the switching circuit 130 may include a control terminal and a switch. The switch may be configured to electrically connect the plurality of electrodes 110 and the contact impedance detection circuit 120. The control terminal may be configured to control on and off of the switch. In some embodiments, the switching circuit 130 may include a single-channel switch to achieve the function of selecting the plurality of electrodes 110. In some embodiments, a count of channels of the single-channel switch may be related to a count of the plurality of electrodes 110, and a count of channels expected to be conducted may be related to a count of electrodes 110 expected to be conducted with the contact impedance detection circuit 120 at the same time. For example, if 8 electrodes 110 are provided, the switching circuit 130 may include a single-channel switch such as an 8-to-1 switch, an 8-to-2 switch, or an 8-to-4 switch, etc. The switching circuit 130 may select one electrode 110 from the 8 electrodes to be connected with the contact impedance detection circuit 120 at the same time using the 8-to-1 switch. Similarly, the switching circuit 130 may select two electrodes 110 (e.g., the two electrodes attached to the same muscle) from the 8 electrodes to be connected with the contact impedance detection circuit 120 at the same time using the 8-to-2 switch.

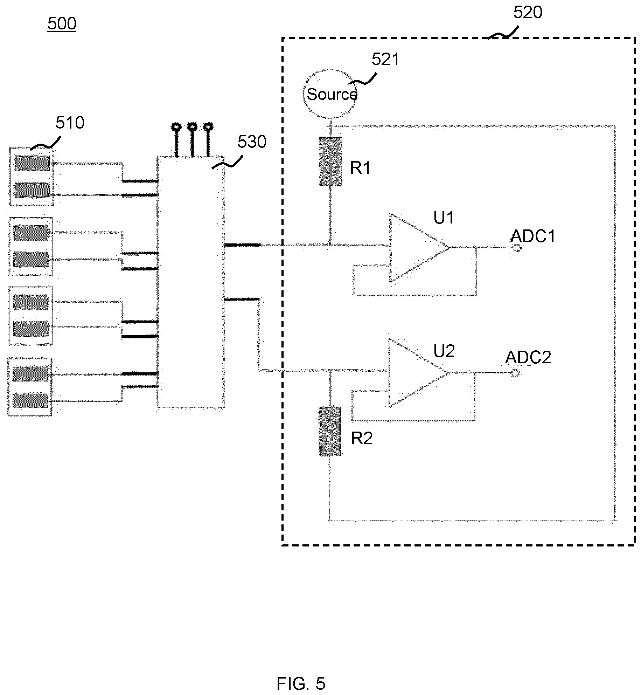

In some embodiments, the switching circuit 130 may include a dual-channel switch to implement the function of selecting the plurality of electrodes 110 and the plurality of contact impedance detection circuits 120. Similar to the single-channel switch, in some embodiments, a count of channels corresponding to the plurality of electrodes 110 in the dual-channel switch may be related to the count of the plurality of electrodes 110, a count of channels corresponding to the plurality of contact impedance detection circuits 120 in the dual-channel switch may be related to a count of contact impedance detection circuits 120, and a count of channels expected to be conducted may be related to a count of electrodes 110 expected to be conducted with the contact impedance detection circuits 120 at the same time. Merely by way of example, if 8 electrodes 110 and 4 contact impedance detection circuits 120 are provided, the switching circuit 130 may include a dual-channel switch that selects 2 electrodes from the 8 electrodes 110 and selects 1 contact impedance detection circuit 120 from the 4 contact impedance detection circuits 120. More descriptions regarding specific implementation of the dual-channel switch may be found elsewhere in the present disclosure (e.g., FIG. 5 and the related descriptions thereof), which are not repeated here.

In some embodiments, the switching circuit 130 may control the plurality of electrodes 110 to collect the physiological signals and measure the contact impedances corresponding to the plurality of electrode 110 in a time-sharing manner. In some embodiments, the switching circuit 130 may control the plurality of electrodes 110 to be in the conduction state with the contact impedance detection circuit 120 and a gain circuit (used to process the physiological signals) at different times. If the plurality of electrodes 110 are in the conduction state with the contact impedance detection circuit 120, the contact impedance detection circuit 120 may obtain and process the detection signals corresponding to the plurality of electrode 110 to obtain the contact impedance information corresponding to the plurality of electrodes 110. If the plurality of electrodes 110 are in the conduction state with the gain circuit, the plurality of electrodes 110 may collect the physiological signals, and the gain circuit may process the collected physiological signals to obtain information (e.g., muscle state information) reflecting the physiological state of the human body.

In this way, by controlling the plurality of electrodes 110 to asynchronously collect the physiological signals and measure the contact impedances through the switching circuit 130, the detection signals and the physiological signals can be separated, thereby preventing a mutual interference of the signals and further improving the quality of the physiological signals.

It should be noted that the physiological signals and the detection signals may also be processed synchronously. In order to distinguish the physiological signals from the detection signals, the physiological signals and the detection signals may be extracted respectively by using a filter based on a frequency difference between the physiological signals the detection signals, so as to subsequently evaluate the physiological signals and the detection signals. For example, the physiological signals after the analog-to-digital conversion may be extracted using a low-pass filter, and the detection signals after the analog-to-digital conversion may be extracted using a highpass filter.

FIG. 2 is a structural block diagram illustrating an exemplary contact impedance detection circuit according to some embodiments of the present disclosure. As shown in FIG. 2, in some embodiments, the contact impedance detection circuit 120 may include: an excitation source 121, a voltage-dividing impedance 122, and an analog-to-digital converter 123. The excitation source 121 may be connected with the voltage-dividing impedance 122, and configured to provide an excitation signal to each of the plurality of electrodes 110 to generate a detection signal reflecting a contact impedance between each of the plurality of electrodes and a human body. The voltage-dividing impedance 122 may be connected with each of the plurality of electrodes 110, and configured to form a detection signal corresponding to each electrode by performing a voltage division on the excitation source 121. The analog-to-digital converter 123 may be connected with the voltage-dividing impedance 122, and configured to perform an analog-to-digital conversion on the detection signal corresponding to each electrode.

The excitation source 121 refers to a circuit element that provides electrical energy. In some embodiments, the excitation source 121 may be a current source or a voltage source. In some embodiments, the excitation source 121 may provide an excitation signal to generate the detection signal reflecting the contact impedance between each of the plurality of electrodes 110 and the human body. Merely by way of example, the excitation signal may be understood as forming a closed loop after flowing through the human body via the plurality of electrodes 110. In this case, the detection signal may correspond to the contact impedance between each of the plurality of electrodes 110 and the human body, and a voltage division of the impedance of the human body in the closed loop. The higher the intensity of the excitation source 121, the easier the detection signal is to be detected. It should also be noted that the setting of the intensity of the excitation source 121 also needs to consider the safety voltage and current of the human body to ensure the safety of the human body. The intensity of the excitation source 121 should not be too high. In some embodiments, a current intensity of the excitation source 121 may be less than 1 mA. In some embodiments, the current intensity of the excitation source 121 may be less than 100 µA. In some embodiments, the current intensity of the excitation source 121 may be less than 10 µA. In some embodiments, a voltage intensity of the excitation source 121 may be within a range of 10 µV-3.3 V. In some embodiments, the voltage intensity of the excitation source 121 may be within a range of 100 mV-9 V.

In some embodiments, the excitation source 121 may be a DC excitation source or an AC excitation source. If a physiological signal and a detection signal are processed in a time-sharing manner, the physiological signal and the detection signal may be unlikely to interfere with each other. In this case, the excitation source 121 may be the DC excitation source or the AC excitation source. If the physiological signal and the detection signal are not processed in a time-sharing manner, in order to avoid a mutual interference between the physiological signal and the detection signal, a frequency of the AC excitation source may be set such that a frequency of the detection signal may be different from a frequency of the physiological signal, and the detection signal and the physiological signal may be extracted separately according to a frequency difference between the detection signal and the physiological signal. In some embodiments, the frequency of the excitation source 121 may be set in a frequency range that is less affected by an external interference (e.g., a power frequency interference) and has a less interference with the physiological signal. For example, in some embodiments, the excitation source 121 may be the DC excitation source or the AC excitation source with a frequency not less than 0.1 Hz.

In some embodiments, one excitation source 121 may be provided, and configured to provide an excitation signal to the plurality of electrodes 110 (e.g., two electrodes 110 attached to the same muscle) by connecting the plurality of electrodes 110 in parallel. In some embodiments, a plurality of excitation sources 121 may be provided, and each of the plurality of excitation sources 121 may be connected with one or more electrodes 110 to provide the excitation signal to each electrode 110. It should be noted that a plurality of contact impedance detection circuits 120 may also share one excitation source 121. By connecting the plurality of electrodes 110 in parallel, the excitation source 121 may provide the excitation signal to all the electrodes 110.

In some embodiments, if the excitation source 121 is the AC excitation source, the excitation source 121 may generate a plurality of excitation signals with different frequencies simultaneously, and may provide excitation signals with the same frequency or different frequencies to the plurality of electrodes 110, thereby generating detection signals with different frequencies.

For example, the excitation source 121 may provide excitation signals of different frequencies to the plurality of electrodes 110 to collect different detection signals. The plurality of electrodes 110 may have a large distance (e.g., greater than or equal to 5 cm) therebetween, such that the closed loop through which the excitation signals flow may pass through different human tissues. In this way, the detection signals of different frequencies corresponding to the plurality of electrodes 110 may be used to reflect body composition information of the human body, such as a body fat rate, a bone mineral density (BMD), a body fluid content, etc.

In some embodiments, the excitation source 121 may provide a second excitation signal different from the excitation signal to generate a second detection signal. The second detection signal may reflect impedance information on a closed loop formed between each of the plurality of electrodes and the human body at another frequency (i.e., a frequency different from the frequency of the excitation signal), including the contact impedance between each of the plurality of electrodes and the human body and an impedance of a human tissue in the closed loop. In some embodiments, the body composition information of the human body may be determined using a first detection signal generated by a first excitation signal (e.g., the above mentioned excitation signal) and the second detection signal generated by the second excitation signal.

In some embodiments, the excitation signal may be a voltage signal or a current signal. In some embodiments, the excitation signal may be a DC excitation signal or an AC excitation signal, such that a frequency of the generated detection signal may be related to the frequency of the excitation signal.

In some embodiments, the voltage-dividing impedance 122 may be disposed between the excitation source 121 and each of the plurality of electrodes 110. Since the plurality of electrodes 110 and the voltage-dividing impedance 122 are in the same loop, the detection signal may correspond to the contact impedance between each of the plurality of electrodes 110 and the human body, and the voltage division of the impedance of the human body in the closed loop, or correspond to a divided voltage of the voltage-dividing impedance in the closed loop. Since a magnitude of the detection signal is affected by the contact impedance between each of the plurality of electrodes 110 and the human body, the magnitude of the detection signal may reflect a magnitude of the contact impedance, and thus may reflect the quality of the physiological signal. For example, the greater the intensity of the detection signal corresponding to a certain electrode 110, the greater the corresponding contact impedance, which means that the quality of the physiological signal collected by the electrode is poor; conversely, the smaller the intensity of the detection signal, the smaller the corresponding contact impedance, which means that the quality of the physiological signal collected by the electrode is good.

In some embodiments, a plurality of voltage-dividing impedances 122 may be provided, and each of the plurality of electrodes 110 may be connected with one of the plurality of voltage-dividing impedances 122. In some embodiments, one voltage-dividing impedance 122 may be provided, and connected with each of the plurality of electrodes 110 in a conduction state through the switching circuit 130. In some embodiments, a count of voltage-dividing impedances 122 may be determined according to a count of electrodes 110 in the conduction state. Merely by way of example, if two electrodes 110 synchronously are in the conduction state with the contact impedance detection circuit 120, two voltage-dividing impedances 122 may be provided to synchronously be in the conduction state with the two electrodes 110. If the two electrodes 110 are in the conduction state with the contact impedance detection circuit 120 in a time-sharing manner, one voltage-dividing impedance 122 may be provided, and may be connected with one of the two electrodes 110 in the conduction state.

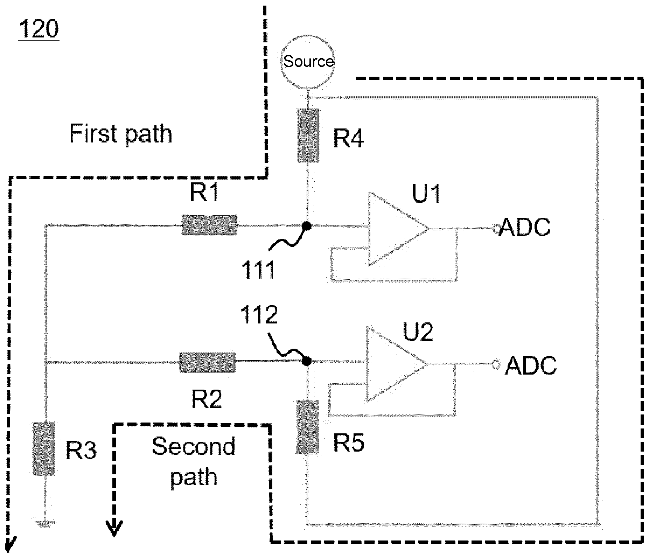

FIG. 3 is a schematic diagram illustrating an exemplary signal measurement circuit according to some embodiments of the present disclosure. As shown in FIG. 3, the plurality of electrodes 110 may include a first electrode 111 and a second electrode 112. The voltage-dividing impedance 122 may include a first voltage-dividing impedance R4 and a second voltage-dividing impedance R5. After the excitation source 121 (an excitation source (Source) shown in FIG. 3) sends an excitation signal, the excitation signal may flow through the first voltage-dividing impedance R4, and flow to a human body through the first electrode 111 after a voltage division is performed by the first voltage-dividing impedance R4, and finally flow to a reference ground electrode to form a first path. The first voltage-dividing impedance R4 may perform the voltage division on the excitation signal to form a detection signal corresponding to the first electrode 111.

Similarly, after the excitation source 121 sends the excitation signal, the excitation signal may flow through the second voltage-dividing impedance R5, and flow to the human body through the second electrode 112 after the voltage division is performed by the second voltage-dividing impedance R5, and finally flow to the reference ground electrode to form a second path. The second voltage-dividing impedance R5 may perform the voltage division on the excitation signal to form a detection signal corresponding to the second electrode 112. A contact impedance R1 refers to a contact impedance between the first electrode 111 and the human body. A contact impedance R2 refers to a contact impedance between the second electrode 112 and the human body. An impedance R3 may include an impedance of the human body and an impedance between the reference ground electrode and the human body.

In some embodiments, the first path and the second path may have a certain degree of symmetry. Specifically, the first voltage-dividing impedance R4 and the second voltage-dividing impedance R5 may have the same parameters. If a plurality of excitation sources 121 are provided, the plurality of excitation sources 121 may have the same parameters. For example, one or more excitation sources 121 may generate excitation signals with the same frequency, and the first voltage-dividing impedance R4 and the second voltage-dividing impedance R5 may have the same impedance under the excitation of the one or more excitation sources 121. In some embodiments, by guaranteeing the symmetry of the first path and the second path, a difference between the detection signal corresponding to the first electrode 111 and the detection signal corresponding to the second electrode 112 may be mainly derived from a difference in the contact impedance between the first electrode 111 and the human body and the contact impedance between the second electrode 111 and the second electrode 112. In this way, by comparing the difference between the detection signals, whether an attachment state between the two electrodes and the human body is good can be reflected, and whether the physiological signals collected by the two electrodes meet the requirements can be reflected.

In some embodiments, the contact impedance detection circuit 120 and the gain circuit may be simultaneously in the conduction state with the plurality of electrodes 110. In this case, if a resistance value of the first voltage-dividing impedance R4 and a resistance value of the second voltage-dividing impedance R5 are too small, a subsequent input impedance may be too small due to the parallel connection, resulting in the intensity of the collected physiological signal being too small to be monitored. For example, the physiological signal may be covered by various noises (including an aliasing noise, a thermal noise, etc.), which may cause the system not to work properly. If the resistance value of the first voltage-dividing impedance R4 and the resistance value of the second voltage-dividing impedance R5 are too large, the divided voltage of the excitation signal may be too large, making the intensity of the detection signal too small to reflect the contact impedance between each of the plurality of electrodes and the human body.

In some embodiments, if the excitation source 121 is a DC excitation source, the voltage-dividing impedance 122 may include a resistor, so as to perform a proper voltage division on the DC excitation source to prevent an excessive voltage division due to excessive resistance value of the voltage-dividing impedance 122, thereby ensuring that the intensity of the detection signal is large and capable of reflecting the contact impedance between each of the plurality of electrodes and the human body.

In some embodiments, if the excitation source 121 is an AC excitation source, the voltage-dividing impedance 122 may include the resistor and/or a capacitor. If the voltage-dividing impedance 122 is the capacitor, different impedances may be provided at different frequencies. In this way, at a frequency of the excitation signal (i.e., a relatively high frequency), the voltage-dividing impedance 122 may have a relatively small impedance, thereby reducing the voltage division of the excitation signal, and making the detection signal better reflect the contact impedance between each of the plurality of electrodes and the human body; and at a relatively low frequency corresponding to most physiological signals, the voltage-dividing impedance 122 may have a relatively large impedance, avoiding the subsequent reduction of an input impedance due to the parallel connection, such that the physiological signals can be more effectively picked up and processed by a subsequent circuit.

In some embodiments, the contact impedance detection circuit 120 and the gain circuit may be in the conduction state with the plurality of electrodes 110 in a time-sharing manner. In some embodiments, when the contact impedance detection circuit 120 is in the conduction state with the plurality of electrodes 110, the gain circuit may be disconnected from the plurality of electrodes 110, or when the gain circuit is in the conduction state with the plurality of electrodes 110, the contact impedance detection circuit 120 may be disconnected from the plurality of electrodes 110. In some embodiments, the gain circuit may be in the conduction state with the plurality of electrodes 110 for a long time, and only the conduction and disconnection of the contact impedance detection circuit 120 and the plurality of electrodes may be switched. In other words, the gain circuit may not be disconnected from the plurality of electrodes. Only when the contact impedance information needs to be collected, the contact impedance detection circuit 120 may be controlled to be in the conduction state with the plurality of electrodes 110 to collect the detection information corresponding to the contact impedance. When the collection is not required, the contact impedance detection circuit 120 may be controlled to be disconnected from the plurality of electrodes 110, saving the switching times of the switching circuit 130. In the case of time-sharing conduction, the contact impedance detection circuit 120 may not affect the gain circuit for processing the physiological signals, i.e., the voltage-dividing impedance 122 may not be connected in parallel with an input end of the gain circuit. In this case, even if the voltage-dividing impedance 122 uses a smaller impedance value, a good quality detection signal may be obtained. In some embodiments, if the excitation source 121 is a 3.3V DC, the voltage-dividing impedance 122 may select a resistor of 1 MΩ - 10MΩ. If the excitation source 121 is an AC, the voltage-dividing impedance 122 may select a capacitor of 1 pF-50 pF.

In some embodiments, the analog-to-digital converter 123 may sample the detection signal to generate a digital signal corresponding to the detection signal. In some embodiments, if the excitation source 121 is the AC excitation source, a sampling rate of the analog-to-digital converter 123 may be set in combination with the frequency of the excitation source 121. Specifically, in order to sample the detection signal, the sampling rate of the analog-to-digital converter 123 may be greater than twice the frequency of the excitation source 121. In order to reduce errors, in some embodiments, the sampling rate of the analog-to-digital converter 123 may be 4 times or more of the frequency of the excitation source 121. It should be noted that since the signal measurement device 100 has a large number of channels, most circuits are difficult to support extremely high sampling rates of the plurality of channels, so a single-channel sampling rate and the frequency of the excitation source 121 should not be too high. For example, the frequency of the excitation source 121 may be 1 kHz, and the single-channel sampling rate may be 8 kHz.

In some embodiments, different analog-to-digital converters may be selected according to the resolution requirements of contact impedance detection. For example, under the condition of 3.3V power supply, the accuracy of a 12-bit analog-to-digital converter may reach 0.8 mV, and the accuracy of a 16-bit analog-to-digital converter may reach 0.05 mV.

In some embodiments, a follower may be coupled between the analog-to-digital converter 123 and the voltage-dividing impedance 122. The follower may be configured to buffer or isolate components with different functions, such as isolating the voltage-dividing impedance 122 and the analog- to-digital converter 123 (an analog-to-digital converter ADC shown in FIG. 3), and to maintain the input of the analog-to-digital converter 123 stable. For example, referring to FIG. 3, a non-inverting input end of a follower U1 may be connected with the first voltage-dividing impedance R4 for receiving the detection signal, and an inverting input end of the follower U1 may be connected with an output end of the follower U1. A non-inverting input end of a follower U2 may be connected with the second voltage-dividing impedance R5 for receiving the detection signal, and an inverting input end of the follower U2 may be connected with an output end of the follower U2.

In some embodiments, the structure (e.g., the excitation source, the voltage-dividing impedance, the analog-to-digital converter, etc.) of the contact impedance detection circuit 120 may also be configured differently according to different time-sharing measurement manners.

In some embodiments, if the switching circuit 130 controls the two electrodes 110 attached to the same muscle to synchronously to be in the conduction state with the contact impedance detection circuit 120, i.e., the two electrodes 110 synchronously collect the detection signals, the contact impedance detection circuit 120 may include the excitation source 121, two voltage-dividing impedances 122, and two analog-to-digital converters 123. The excitation source 121 may provide an excitation signal to the two electrodes 110 attached to the same muscle to generate the detection signal reflecting the contact impedance between each of the two electrodes 110 and the human body. The two voltage-dividing impedances 122 may be respectively coupled between the excitation source 121 and each of the two electrodes 110, and configured to perform a voltage division on the excitation source 121 to form the detection signal. The two analog-to-digital converters 123 may be configured to perform an analog-to-digital conversion on the detection signals corresponding to the two electrodes 110.

For example, the switching circuit 130 may control a first electrode and a second electrode attached to a first muscle to remain in the conduction state with the contact impedance detection circuit 120 at a first time. Each voltage-dividing impedance 122 of the contact impedance detection circuit 120 may be connected with the first electrode and the second electrode. At a second time, the switching circuit may disconnect the contact impedance detection circuit 120 from the first electrode and the second electrode, and control a third electrode and a fourth electrode attached to a second muscle to be in the conduction state with the contact impedance detection circuit 120, so as to obtain detection signals corresponding to the third electrode and the fourth electrode, respectively.

In some embodiments, the switching circuit 130 may control the two electrodes 110 attached to the same muscle to be in the conduction state with the contact impedance detection circuit 120 at different times, i.e., in the scenario where the two electrodes 110 asynchronously collect the detection signals, the contact impedance detection circuit 120 may include the excitation source 121, the voltage-dividing impedance 122, and the analog-to-digital converter 123. The excitation source 121 may be configured to provide an excitation signal to a certain conducted electrode 110 to generate a detection signal reflecting the contact impedance between the electrode 110 and the human body. The voltage-dividing impedance 122 may be coupled between the excitation source 121 and the conducted electrode 110, and configured to perform the voltage division on the excitation source 121 to form the detection signal. The analog-to-digital converter 123 may be configured to perform the analog-to-digital conversion on the detection signal corresponding to the conducted electrode 110.

For example, taking two electrodes 110 (e.g., the first electrode and the second electrode) as an example, the switching circuit 130 may control the first electrode to be in the conduction state with the contact impedance detection circuit at the first time to obtain the detection signal corresponding to the first electrode. The switching circuit 130 may switch the electrode at the second time, and control the second electrode to be in the conduction state with the contact impedance detection circuit to obtain the detection signal corresponding to the second electrode.

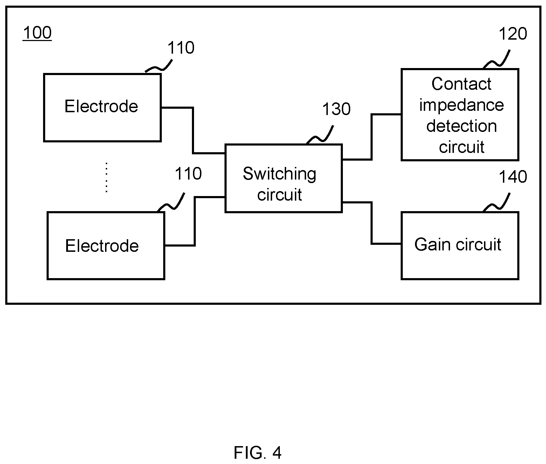

FIG. 4 is a structural block diagram illustrating an exemplary signal measurement circuit according to other embodiments of the present disclosure. In some embodiments, the signal measurement circuit 100 may further include a gain circuit 140. The gain circuit 140 may be configured to process physiological signals. The gain circuit 140 and the contact impedance detection circuit 120 may be in a conduction state with a certain electrode 110 at different times, or be in the conduction state with different electrodes 110 at the same time.

In some embodiments, the gain circuit 140 may be a signal processing circuit for amplifying a signal. In some embodiments, the gain circuit 140 may be configured to provide a gain for the physiological signal to improve an intensity and a signal-to-noise ratio of the physiological signal and ensure the quality of the signal. In some embodiments, the gain circuit 140 and the contact impedance detection circuit 120 may be in the conduction state with the plurality of electrodes 110 at different times, respectively, thereby controlling the plurality of electrodes 110 to asynchronously collect the physiological signals and measure a contact impedance through the switching circuit 130, isolating the detection signals and the physiological signals, preventing mutual interference of the signals, and further improving the quality of the physiological signals.

In some embodiments, one gain circuit 140 may be provided. The gain circuit 140 may be connected with one or more electrodes 110. The switching circuit 130 may control the conduction state between the gain circuit 140 and the plurality of electrodes 110. In some embodiments, a plurality of gain circuits 140 may be provided. The switching circuit 130 may select the plurality of electrodes 110 and the plurality of gain circuits 140 corresponding to the plurality of electrodes 110 to be in the conduction state. A conduction mode between the gain circuit 140 and the plurality of electrodes 110 may be similar to a conduction mode between the contact impedance detection circuit 120 and the plurality of electrodes 110. The specific implementation mode may be found in FIG. 1 and the related descriptions thereof.

In some embodiments, the gain circuit 140 may provide the gain for full-band signals, and may also provide the gain for partial-band signals. Taking an electromyographic signal as an example, most of the electromyographic signals are mainly distributed within a frequency band of 10 Hz-850 Hz, and the gain circuit 140 may provide the gain for the electromyographic signals within the frequency band of 10 Hz-850 Hz to avoid providing the gain for other frequency bands (e.g., frequency bands except the frequency band of 10 Hz-850 Hz) , thereby suppressing the interference of noise on the electromyographic signal and improving the signal-to-noise ratio of the electromyographic signal.

In some embodiments, the gain may be expressed as a degree of amplification of the electromyographic signal. For example, the greater the gain provided by the gain circuit 140, the greater the multiple of the electromyographic signal is amplified. Conversely, the smaller the gain provided by the gain circuit 140, the smaller the multiple of the electromyographic signal is amplified.

In some embodiments, a magnitude of the gain may be set according to an actual signal intensity and an expected signal intensity. In some embodiments, the magnitude of the gain may be set according to the actual signal intensity and the expected signal intensity of the electromyographic signal and a detection signal. For example, since the actual signal intensity of the electromyographic signal is about 10 µV or less, and the resolution of a commonly used 12-bit analog-to-digital converter is about 0.8 mV (i.e., the expected signal intensity), a gain of more than 100 times, which may reach 1000 times, may be provided using the gain circuit 140, such that the signal intensity of the electromyographic signal after the gain is provided may reach a range of 1 mV-1.65 V.

Since there are a plurality of interference signals in the process of signal measurement, such as motion artifacts, power frequency and harmonics thereof, aliasing noise, etc. On the one hand, if too much gain is provided for the interference signals, the interference signals after the gain is provided may exceed a measurement range of the circuit, which may lead to circuit saturation and data loss, making it difficult to recover the data through an algorithm. On the other hand, if too much gain is provided for a high-frequency noise, due to the limitation of the sampling rate, the high-frequency noise exceeding the sampling rate may be aliased, increasing the noise floor and causing the high-frequency noise to overlap with the electromyographic signal, which makes it difficult to distinguish and process the high-frequency noise and the electromyographic signal.

In some embodiments, a gain at 10 Hz may be represented as a gain of motion artifacts, and a ratio of a gain at 100 Hz to the gain at 10 Hz provided by the gain circuit 140 may be denoted as a first signal-to-noise ratio. In order to improve the gain signal-to-noise ratio of the gain circuit 140, the first signal-to-noise ratio may be set to be not less than 4. In some embodiments, a gain at 50 Hz may be represented as a gain of power frequency noise, and a ratio of the gain at 100 Hz to the gain at 50 Hz provided by the gain circuit 140 may be denoted as a second signal-to-noise ratio. In order to improve the gain signal-to-noise ratio of the gain circuit 140, the second signal-to-noise ratio may not be less than 2. In some embodiments, since the generation of the aliasing noise is related to the sampling rate, a 1/2 frequency of the sampling rate of the analog-to-digital converter may be represented as a frequency of the aliasing noise, which is also referred to as a first frequency. For example, if the sampling frequency is 4 kHz, the frequency of the aliasing noise may be 2 kHz. A ratio of the gain at 100 Hz to the gain at the first frequency provided by the gain circuit 140 may be denoted as a third signal-to-noise ratio (i.e., the aliasing signal-to-noise ratio). In order to improve the gain signal-to-noise ratio of the gain circuit 140, the third signal-to-noise ratio may not be less than 10.

It should be noted that the gain signal-to-noise ratio may also be used to measure the quality of the gain circuit. The higher the gain signal-to-noise ratio of the signal measurement device, the better the intensity and the quality of the obtained electromyographic signal, and the higher the quality of the gain circuit.

In some embodiments, the gain circuit 140 may include multi-stage amplification subcircuits which are configured to perform multi-stage amplification processing on an input signal (e.g., the physiological signal) to achieve the function of providing the gain. In some embodiments, the amplification subcircuits may be independently set in the signal measurement circuit 100, and may also be set as a component in a circuit with other functions (e.g., a filtering circuit, etc.), and set as a whole with the circuit with other functions, thereby providing the gain while achieving other functions. In some embodiments, different stages of amplification subcircuits of the multi-stage amplification subcircuits may have different amplification gains for the input signal. For example, a gain provided by an amplification subcircuit at a front stage may be smaller than a gain by an amplification subcircuit at a rear stage.

In some embodiments, the gain circuit 140 may also be provided with a highpass filter to filter out low-frequency noises such as the motion artifacts and the power frequency noise. In some embodiments, the gain circuit 140 may also be provided with a low-pass filter to filter out high-frequency interference signals, such as the aliasing noise, and other high-frequency noises. Cutoff frequencies of the high-frequency filter and the low-pass filter may be set according to a frequency of the physiological signal.

In some embodiments, since values of contact impedances between the human body and the plurality of electrode may be small, the intensity of the detection signals generated by the voltage division may be low, and the detection signals may be difficult to be reorganized and processed subsequently. The gain circuit 140 may also be configured to provide a gain for the detection signal to increase the intensity of the detection signal, ensure that the detection signal is be accurately reorganized and processed subsequently. In some embodiments, the gain circuit 140 may provide the gain for the physiological signal and the detection signal, respectively, in different time periods.

In some embodiments, the signal measurement circuit 100 may further include a processing circuit configured to determine parameter information reflecting an attachment state of each of the plurality of electrodes 110 to the human body according to the contact impedance of each of the plurality of electrodes 110.

In some embodiments, the processing circuit may be a module with a certain signal processing function in terms of the structure of the processing circuit. In some embodiments, the parameter information may include the impedance information between each of the plurality of electrodes 110 and the human body, which may reflect the attachment state of each of the plurality of electrodes 110 to the human body, and further reflect a current wearing state of a wearable device, a fit degree of size, etc.

In some embodiments, the processing circuit may evaluate the attachment state of each of the plurality of electrodes 110 to the human body based on the parameter information and an amplitude range of the parameter information. For example, if the parameter information is outside a target amplitude range, it means that the quality of the collected physiological signal is very poor, and it is difficult to use the physiological signal for physiological monitoring subsequently, so it is necessary to output anomaly reminder information to remind the user to check and adjust in time. If the parameter information is within the target amplitude range, it is determined that the current wearing state of the wearable device is good, or the fit degree of size of the wearable device is suitable, or the user is in a fully warmed-up state, or the skin where a current electrode contacts with the human body is in a relatively moist state. The type of the parameter information and the limitation of the target amplitude range may be adjusted according to the measurement requirements.

In some embodiments, if the quality of the physiological signal is very poor, the processing circuit may also correct or compensate the physiological signal using a signal processing algorithm, thereby ensuring the quality or accuracy of the physiological signal. In some embodiments, the signal processing algorithm may include one or more of the following algorithms including signal filtering, wavelet transform, machine learning, etc.

In some embodiments, the signal measurement circuit 100 may be applied to the wearable device. In some embodiments, the wearable device may include a smart bracelet, smart shoes and socks, smart glasses, a smart helmet, a smart watch, smart clothing, a smart backpack, a smart accessory, or the like, or any combination thereof. Taking the smart clothing (e.g., fitness clothing or sportswear) as an example, the signal measurement circuit 100 may obtain physiological signals collected by any two or two electrodes with a specific association e.g., the two electrodes attached to the same muscle to receive the electromyographic signal of the same muscle), and evaluate the quality of the physiological signals based on a difference of the physiological signals collected by the any two or two electrodes with the specific association, or evaluate the attachment state of the any two or two electrodes with the specific association to the human body.

In some embodiments, the signal measurement circuit 100 may also perform wearing state analysis, warm-up state analysis, analysis on the fit degree of size, clothing life analysis, motion monitoring, skin state analysis, body composition analysis, etc., based on evaluation results of the physiological signals.

A specific implementation of time-sharing measurement is described in detail below with reference to an exemplary signal measurement circuit 500.

FIG. 5 is a schematic structural diagram illustrating an exemplary signal measurement circuit according to some embodiments of the present disclosure. As shown in FIG. 5, the signal measurement circuit 500 may include eight electrodes 510, a contact impedance detection circuit 520, and a switching circuit 530. The contact impedance detection circuit 520 may include an excitation source 521 (e.g., an excitation source (Source) shown in FIG. 5), voltage-dividing impedances R1-R2, followers U1-U2, and analog-to-digital converters ADC1-ADC 2. Every two of the eight electrodes 510 may be used as an electrode group to be attached to the same muscle, and the two electrodes 510 may be configured to measure physiological signals of the muscle. The eight electrodes 510 may be respectively coupled with the switching circuit 530. One end of each of the voltage-dividing impedances R1-R2 may be coupled with the excitation source 521, and the other end of each of the voltage-dividing impedances R1-R2 may be coupled with the switching circuit 530. The voltage-dividing impedances R1-R2 may perform a voltage division on the excitation source 521 to generate a detection signal reflecting a contact impedance between each electrode 510 and the human body. A coupling end of each of the voltage-dividing impedances R1-R2 with and the switching circuit 530 may also be connected with each of the analog-to-digital converters. The analog-to-digital converters may be configured to convert the detection signal into a digital signal for subsequent processing by a device, such as calculating the contact impedance between the electrode and the human body, recognizing an attachment state of the electrode to the human body, etc. The followers U1-U2 may be arranged between the voltage-dividing impedances R1- R2 and the analog-to-digital converters. The followers U1-U2 may be configured to stabilize the detection signals input to the analog-to-digital converters ADC1-ADC2.

In some embodiments, the switching circuit 530 may control two electrodes 510 of the eight electrodes 510 (i.e., select 2 electrodes from 8 electrodes) to simultaneously be in the conduction state with the contact impedance detection circuit 520, such that two detection signals may be simultaneously generated under the action of the excitation source 521, and the contact impedance detection circuit 520 may receive the detection signals reflecting the contact impedances between the two electrodes 510 and the human body, and perform an analog-to-digital conversion on the two detection signals. In some embodiments, the switching circuit 530 may include an 8-to-2 switch to achieve the above function of selecting two electrodes 510 from the eight electrodes 510 to simultaneously be in the conduction state with the contact impedance detection circuit 520.

In some embodiments, the switching circuit 530 may control the two electrodes 510 of the eight electrodes 510 to be in the conduction state with the contact impedance detection circuit 520 at different times, such that two detection signals may asynchronously generated under the action of the excitation source 521. That is, at the same time, only one electrode 510 (8-to-1) of the eight electrodes 510 may be in the conduction state with the contact impedance detection circuit 520. The contact impedance detection circuit 520 may successively receive the detection signals reflecting the contact impedances between the two electrodes 510 and the human body, and perform the analog-to-digital conversion on the two detection signals. In some embodiments, the switching circuit 530 may include the 8-to-2 switch to achieve the above function of controlling the two electrodes 510 of the eight electrodes 510 to be in the conduction state with the contact impedance detection circuit 520 at different times. In some embodiments, the switching circuit 530 may also include an 8-to-1 switch to achieve the function of controlling only one electrode 510 of the eight electrodes 510 to remain in the conduction state with the contact impedance detection circuit 520 at the same time.

In some optional embodiments, the switching circuit 530 may also control a portion of the eight electrodes 510 to remain in the conduction state with the contact impedance detection circuit 520 and a gain circuit (not shown in FIG. 5) at different times, so as to achieve asynchronous collection of the physiological signals and detection of the contact impedances.

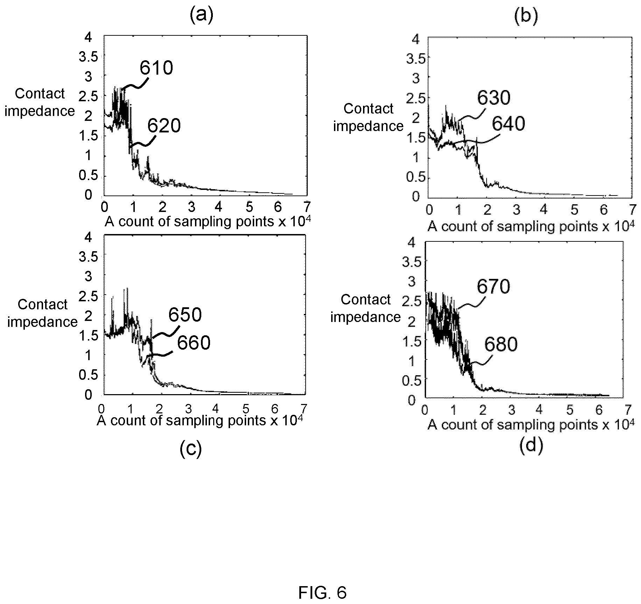

FIG. 6 is a schematic diagram illustrating exemplary curves of contact impedances corresponding to a plurality of electrodes during exercise of a user according to some embodiments of the present disclosure. A horizontal axis represents a count of sampling points, and a vertical axis represents a magnitude of the contact impedances corresponding to the plurality of electrodes. FIG. 6(a) includes a curve 610 and a curve 620 of contact impedances corresponding to two electrodes attached to a left pectoral muscle. FIG. 6(b) includes a curve 630 and a curve 640 of contact impedances corresponding to two electrodes attached to left triceps. FIG. 6(c) includes a curve 650 and a curve 660 of contact impedances corresponding to two electrodes attached to left biceps. FIG. 6(d) includes a curve 670 and a curve 680 of contact impedances corresponding to two electrodes attached to a left oblique abdominal muscle.

As shown in FIGs. 6(a)-6(d), a difference between the contact impedances of the two electrodes attached to different muscles (e.g., an ordinate difference between the curve 610 and the curve 620, an ordinate difference between the curve 630 and the curve 640, an ordinate difference between the curve 650 and the curve 660, and an ordinate difference between the curve 670 and the curve 680) may also be different. As the warm-up progresses (e.g., an increase in the horizontal axis indicates an increase in time), absolute values (e.g., ordinate sizes of the curves 610-680) of the contact impedances may decrease, and the difference between the contact impedances may also decrease. Therefore, it is determined that warm-up can help improve the attachment state of the plurality of electrodes to the human body and improve the quality of the collected physiological signals.

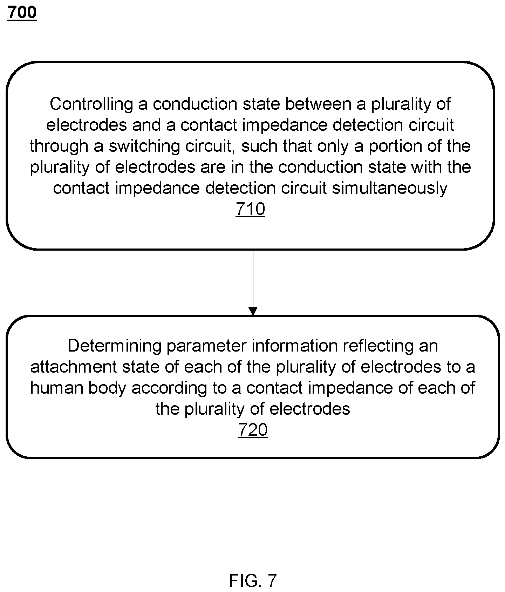

FIG. 7 is a flowchart illustrating an exemplary process for signal measurement according to some embodiments of the present disclosure. In some embodiments, a process 700 may be performed by an aforementioned smart wearable device. The smart wearable device may include a processor, a storage device, and the signal measurement circuit 100 as described above. The storage device may be configured to store computer programs for executing the process 700. The processor may be a portion of a processing circuit of the signal measurement circuit 100, or may be an independently arranged electronic device capable of realizing the function of the processing circuit. More descriptions regarding the signal measurement circuit 100 may be found in FIGs. 1-6 and the related descriptions thereof.

Referring to FIG. 7, in some embodiments, the process 700 may include the following operations.

In 710, a conduction state between a plurality of electrodes and a contact impedance detection circuit may be controlled through a switching circuit, such that only a portion of the plurality of electrodes may be in the conduction state with the contact impedance detection circuit simultaneously. In some embodiments, the operation 710 may be implemented by the switching circuit 130 of the signal measurement circuit 100.

According to the above description of the signal measurement circuit 100, in some embodiments, the plurality of electrodes of the signal measurement circuit 100 may be configured to be attached to the human body to collect physiological signals (e.g., electromyographic signals) of the human body. In the case of setting an excitation source, detection signals reflecting contact impedances between the plurality of electrodes and the human body may also be obtained. If the contact impedance detection circuit and the portion of the plurality of electrodes are in the conduction state, the contact impedance detection circuit may receive the detection signals corresponding to the portion of the electrodes. The contact impedance detection circuit may perform an operation (e.g., a differential operation, and summation) on the detection signals to determine the contact impedances between the human body and the portion of the plurality of electrodes. Another portion of the electrodes may be in the conduction state with the contact impedance detection circuit at another time.

In some embodiments, the plurality of electrodes may include two electrodes attached to the same muscle. Correspondingly, in some embodiments, the switching circuit may control the two electrodes attached to the same muscle to simultaneously be in the conduction state with the contact impedance detection circuit. That is, the contact impedance detection circuit may capture contact impedance information of the two electrodes attached to the same muscle at the same time, thereby avoiding fluctuations in the contact impedances over time and interference from other signals (e.g., detection signals corresponding to other electrodes), and more accurately evaluating the quality of the physiological signals.

In some embodiments, the two electrodes attached to the same muscle may be controlled to be in the conduction state with the contact impedance detection circuit at different times. That is, the contact impedance detection circuit may asynchronously obtain two detection signals and asynchronously detect the contact impedances between the two electrodes and the human body, thereby reducing the burden of processing the detection circuits at the same time, such as reducing the requirements of the system for a sampling frequency in a subsequent process of analog-to-digital conversion. In addition, the asynchronous detection may save the subsequent circuit cost and prevent mutual interference between the two electrodes, thereby further improving the quality of the physiological signals.

In some embodiments, the switching circuit may control the plurality of electrodes to be in the conduction state with the gain circuit and the contact impedance detection circuit at different times, i.e., asynchronously collect the physiological signals and measure the contact impedances, thereby isolating the detection signals and the physiological signals, preventing mutual interference of the signals, and further improving the quality of the physiological signals.

In 720, parameter information reflecting an attachment state of each of the plurality of electrodes to a human body may be determined according to a contact impedance of each of the plurality of electrodes. In some embodiments, the operation 720 may be implemented by the contact impedance detection circuit 120 or a processing circuit of the signal measurement circuit 100.

In some embodiments, the parameter information may include impedance information between each of the plurality of electrodes and the human body, and the attachment state between each of the plurality of electrodes and the human body may be reflected through the parameter information, and then a current wearing state of a wearable device and a fit degree of size may be reflected. In some embodiments, the current wearing state of the wearable device, the fit degree of size, a warm-up state, a clothing life state, motion monitoring, a skin state at a contact point between each of the plurality of electrodes and the human body, one or more pieces of body composition information, or other wear information may also be evaluated based on the parameter information of the attachment state between each of the plurality of electrodes and the human body.

In some embodiments, when the contact impedance of each of the plurality of electrodes is greater than a certain threshold, it may reflect that the electrode is not worn correctly or the user's skin is too dry, such that reminder information may be sent to the user to remind the user to check or adjust the wearing state and the fit degree of size of each of the plurality of electrodes.

In some embodiments, a detection signal reflecting the quality of the physiological signal may also be obtained. An indicator parameter value may be determined by performing digital sampling on the detection signal. The indicator parameter value may be used to reflect the quality of the physiological signal.

In some embodiments, the processing circuit may determine the quality of the physiological signal according to an amplitude fluctuation of the indicator parameter value within a preset time period. For example, if the indicator parameter value is outside a target amplitude range, it may reflect that the quality of the collected physiological signal is very poor, and it is difficult to use the physiological signal for physiological monitoring in the future, so it is necessary to output anomaly reminder information to remind the user to check and adjust in time. In some embodiments, there are many factors that cause the quality of the physiological signal to be very poor, such as the separation of the electrode from the human skin or the occurrence of a lesion in the user's body, etc. The user may check various factors in time according to the anomaly reminder information.

If the indicator parameter value is within a first amplitude range, the processing circuit may output first indication information instructing the user to exercise. The indicator parameter value being within the first amplitude range indicates that the quality of the physiological signal is still poor and it is difficult to accurately reflect the physiological state of the user, so the user is advised to detect or adjust to improve the quality of the physiological signal. The user may exercise according to the first indication information and reduce the contact impedance by warming up, thereby improving the quality of the physiological signal.

If the indicator parameter value is within a second amplitude range, the processing circuit may output second indication information instructing the user to adjust an attachment state of the plurality of electrodes. If the indicator parameter value is within the second amplitude range, it indicates that there may be an anomaly in the attachment state between the plurality of electrodes and the human skin, such as poor contact or incomplete attachment, resulting in poor quality of the physiological signal and difficulty in accurately reflecting the physiological state of the user. In this case, the user may adjust the attachment state of the plurality of electrodes according to the second indication information such that the user may change the magnitude of the contact impedances.

In some embodiments, if the indicator parameter value is low, a movement performed by the user may also cause the amplitude fluctuation of the indicator parameter value. Accordingly, in some embodiments, the movement of the user may be monitored according to the amplitude fluctuation of the indicator parameter value within a preset time period. For example, if the indicator parameter value is less than a preset threshold, and the indicator parameter value has more than a preset count of peaks within the preset time period, it is determined that the user performs the movement within the preset time period.

According to FIG. 6, in some embodiments, the warm-up state of the user may be determined based on the difference or the ratio between the contact impedances of two electrodes attached to the same muscle. In some embodiments, the difference or the ratio between the contact impedances may reflect the warm-up state of the user. Referring to FIG. 6, as time goes by, the smaller the difference or ratio between the contact impedances, the better the warm-up state; conversely, the larger the difference or ratio between the contact impedances, the worse the warm-up state. It should be noted that the contact impedance of each electrode may also reflect the warm-up state of the user individually. For example, the smaller the absolute value of the contact impedance, the better the warm-up state.

It should be noted that, in some embodiments, the detection signals of different frequencies corresponding to the plurality of electrodes 110 may also be used to reflect the body composition information of the human body, such as a body fat rate, a BMD, a body fluid content, and other body composition information.

The beneficial effects that may be brought about by the embodiments of the present disclosure include but are not limited to the following content: (1) the contact impedances between the plurality of electrodes and the human body can be measured by the contact impedance detection circuit, and the attachment state between the plurality of electrodes and the human body can be determined based on the contact impedances, thereby ensuring the quality of the physiological signals collected by the plurality of electrodes; (2) if it is necessary to monitor the attachment state of the plurality of electrodes to different parts of the human body, different portions of electrodes can be selected to be in the conduction state with the contact impedance detection circuit at different times to collect the contact impedances corresponding to different portions electrodes in a time-sharing manner, thereby preventing interference between different electrodes and saving resources of the contact impedance detection circuit.

Having thus described the basic concepts, it may be rather apparent to those skilled in the art after reading this detailed disclosure that the foregoing detailed disclosure is intended to be presented by way of example only and is not limiting. Various alterations, improvements, and modifications may occur and are intended to those skilled in the art, though not expressly stated herein. These alterations, improvements, and modifications are intended to be suggested by this disclosure and are within the spirit and scope of the exemplary embodiments of this disclosure.

Moreover, certain terminology has been used to describe embodiments of the present disclosure. For example, the terms "one embodiment," "an embodiment," and "some embodiments" mean that a particular feature, structure, or feature described in connection with the embodiment is included in at least one embodiment of the present disclosure. Therefore, it is emphasized and should be appreciated that two or more references to "an embodiment" or "one embodiment" or "an alternative embodiment" in various portions of this specification are not necessarily all referring to the same embodiment. Furthermore, the particular features, structures, or features may be combined as suitable in one or more embodiments of the present disclosure.

Furthermore, the recited order of processing elements or sequences, or the use of numbers, letters, or other designations therefore, is not intended to limit the claimed processes and methods to any order except as may be specified in the claims. Although the above disclosure discusses through various examples what is currently considered to be a variety of useful embodiments of the disclosure, it is to be understood that such detail is solely for that purpose and that the appended claims are not limited to the disclosed embodiments, but, on the contrary, are intended to cover modifications and equivalent arrangements that are within the spirit and scope of the disclosed embodiments. For example, although the implementation of various parts described above may be embodied in a hardware device, it may also be implemented as a software only solution, e.g., an installation on an existing server or mobile device.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various embodiments. This method of disclosure, however, is not to be interpreted as reflecting an intention that the claimed subject matter requires more features than are expressly recited in each claim. Rather, claimed subject matter may lie in less than all features of a single foregoing disclosed embodiment.

In some embodiments, numbers describing the number of ingredients and attributes are used. It should be understood that such numbers used for the description of the embodiments use the modifier "about", "approximately", or "substantially" in some examples. Unless otherwise stated, "about", "approximately", or "substantially" indicates that the number is allowed to vary by ±20%. Correspondingly, in some embodiments, the numerical parameters used in the description and claims are approximate values, and the approximate values may be changed according to the required features of individual embodiments. In some embodiments, the numerical parameters should consider the prescribed effective digits and adopt the method of general digit retention. Although the numerical ranges and parameters used to confirm the breadth of the range in some embodiments of the present disclosure are approximate values, in specific embodiments, settings of such numerical values are as accurate as possible within a feasible range.

For each patent, patent application, patent application publication, or other materials cited in the present disclosure, such as articles, books, specifications, publications, documents, or the like, the entire contents of which are hereby incorporated into the present disclosure as a reference. The application history documents that are inconsistent or conflict with the content of the present disclosure are excluded, and the documents that restrict the broadest scope of the claims of the present disclosure (currently or later attached to the present disclosure) are also excluded. It should be noted that if there is any inconsistency or conflict between the description, definition, and/or use of terms in the auxiliary materials of the present disclosure and the content of the present disclosure, the description, definition, and/or use of terms in the present disclosure is subject to the present disclosure.

Finally, it should be understood that the embodiments described in the present disclosure are only used to illustrate the principles of the embodiments of the present disclosure. Other variations may also fall within the scope of the present disclosure. Therefore, as an example and not a limitation, alternative configurations of the embodiments of the present disclosure may be regarded as consistent with the teaching of the present disclosure. Accordingly, the embodiments of the present disclosure are not limited to the embodiments introduced and described in the present disclosure explicitly.

## Claims

1. A signal measurement circuit, comprising:
a plurality of electrodes configured to be attached to a human body and collect physiological signals of the human body;
a contact impedance detection circuit electrically connected with the plurality of electrodes, the contact impedance detection circuit being configured to measure a contact impedance between each of the plurality of electrodes and the human body; and
a switching circuit configured to control a conduction state between the plurality of electrodes and the contact impedance detection circuit, such that only a portion of the plurality of electrodes is in the conduction state with the contact impedance detection circuit simultaneously.

2. The signal measurement circuit of claim 1, wherein the plurality of electrodes include two electrodes attached to the same muscle, and the two electrodes attached to the same muscle are synchronously in the conduction state with the contact impedance detection circuit.

3. The signal measurement circuit of claim 1, wherein the plurality of electrodes include two electrodes attached to the same muscle, and the two electrodes attached to the same muscle are in the conduction state with the contact impedance detection circuit at different times.

4. The signal measurement circuit of claim 2, wherein the contact impedance detection circuit includes:
an excitation source configured to provide an excitation signal to the two electrodes attached to the same muscle to generate detection signals each of which corresponds to one of the two electrodes attached to the same muscle, each detection signal reflecting the contact impedance between the corresponding electrode and the human body;
two voltage-dividing impedances, each of the two voltage-dividing impedances being coupled between the excitation source and one of the two electrodes attached to the same muscle to form the corresponding detection signal by performing a voltage division on the excitation source; and
two analog-to-digital converters configured to perform an analog-to-digital conversion on the detection signals, respectively.

5. The signal measurement circuit of claim 3, wherein the contact impedance detection circuit includes:
an excitation source configured to provide an excitation signal to each of the plurality of electrodes to generate a detection signal reflecting the contact impedance between each of the plurality of electrodes and the human body;
a voltage-dividing impedance coupled between the excitation source and each of the plurality of electrodes to form the detection signal by performing a voltage division on the excitation source; and
an analog-to-digital converter configured to perform an analog-to-digital conversion on the detection signal.

6. The signal measurement circuit of claim 4 or 5, wherein the excitation source is a DC excitation source or an AC excitation source with a frequency not less than 0.1 Hz.

7. The signal measurement circuit of claim 4 or 5, wherein the voltage-dividing impedance includes a resistor or a capacitor.

8. The signal measurement circuit of claim 4 or 5, wherein a follower is coupled between the analog-to-digital converter and the voltage-dividing impedance.

9. The signal measurement circuit of claim 1, further comprising a gain circuit configured for processing the physiological signals, wherein the gain circuit and the contact impedance detection circuit are in the conduction state with the plurality of electrodes at different times, respectively.

10. The signal measurement circuit of claim 9, wherein the gain circuit provides a gain for the physiological signals.

11. The signal measurement circuit of claim 10, wherein a ratio of a gain of the gain circuit at 100 Hz to a gain of the gain circuit at 10 Hz is a first signal-to-noise ratio, the first signal-to-noise ratio being not less than 4.

12. The signal measurement circuit of claim 10, wherein a ratio of a gain of the gain circuit at 100 Hz to a gain of the gain circuit at 50 Hz is a second signal-to-noise ratio, the second signal-to-noise ratio being not less than 2.

13. The signal measurement circuit of claim 10, wherein a ratio of a gain of the gain circuit at 100 Hz to a gain of the gain circuit at a first frequency is a third signal-to-noise ratio, the first frequency being a frequency corresponding to 1/2 of a sampling rate of an analog-to-digital converter, and the third signal-to-noise ratio being not less than 10.

14. The signal measurement circuit of claim 1, further comprising a processing circuit configured to determine parameter information reflecting an attachment state of each of the plurality of electrodes to the human body according to the contact impedance of each of the plurality of electrodes.

15. The signal measurement circuit of claim 1, wherein the signal measurement circuit is disposed in a wearable device.

16. A method for signal measurement, comprising:
controlling a conduction state between a plurality of electrodes and a contact impedance detection circuit through a switching circuit, such that only a portion of the plurality of electrodes are in the conduction state with the contact impedance detection circuit simultaneously, wherein the plurality of electrodes are configured to be attached to a human body and collect physiological signals of the human body, and the contact impedance detection circuit is configured to measure a contact impedance between each of the plurality of electrodes and the human body; and
determining parameter information reflecting an attachment state of each of the plurality of electrodes to the human body according to the contact impedance of each of the plurality of electrodes.

17. The method for signal measurement of claim 16, further comprising:
determining a warm-up state of a user based on a difference or a ratio of the contact impedances of two of the plurality of electrodes attached to the same muscle.

18. The method for signal measurement of claim 16, further comprising:
for each of the plurality of electrodes,
obtaining a detection signal reflecting the contact impedance between the electrode and the human body; and
determining an indicator parameter value reflecting a quality of the physiological signal of the human body collected by the electrode by processing the detection signal.

19. The method for signal measurement of claim 18, further comprising:
in response to determining that the indicator parameter value is within a first amplitude range, outputting first indication information indicating a movement of the user.

20. The method for signal measurement of claim 18, further comprising:
in response to determining that the indicator parameter value is within a second amplitude range, outputting second indication information to remind the user to adjust the attachment state of each of the plurality of electrodes.
